# EUROPEAN PATENT APPLICATION

(11) **EP 1 424 318 A1**
(43) Date of publication of application: **02.06.2004**
(21) Application number: 02080677.4
(22) Date of filing: 26.11.2002
(51) Int. Cl.: C07C 2/58, C07C 2/00, C07C 2/56

(54) **Alkylation process using a reactor comprising multiple catalyst beds**

(71) Applicant: Akzo Nobel N.V., 6824 BM Arnhem (NL); ABB LUMMUS GLOBAL INC., Bloomfield New Jersey 07003 (US); Fortum Oil and Gas Oy, 00048 Fortum (FI)
(72) Inventor: Broekhoven, Emanuel Hermanus van, 1141 DN Monnickendam (NL); D'Amico, Vincent James, Glen Ridge, NJ 07028 (US); Jakkula, Juha, 04200 Kerava (FI)
(74) Representative: Schalkwijk, Pieter Cornelis

(57) **Abstract**

The present invention relates to a process for the alkylation of an alkylatable compound with an alkylation agent using a reactor comprising at least two catalyst beds. At least one of these catalyst beds is a liquid fluidized catalyst bed during at least a part of the process.
The process involves the following steps: (a) introducing alkylatable compound into the reactor at or near the most upstream catalyst bed, (b) introducing alkylation agent into said reactor through at least two injection points, (c) reacting a portion of the alkylatable compound with at least a portion of the alkylation agent to produce an alkylate-containing product stream, and (e) circulating at least a portion of the alkylate-containing product stream to the most upstream catalyst bed of said reactor.

## Description

This invention relates to a process for the alkylation of hydrocarbons using a reactor comprising more than one catalyst bed.
Within the framework of the present invention, the term alkylation refers to the reaction of an alkylatable compound, such as an aromatic or a saturated hydrocarbon, with an alkylation agent, such as an olefin. Without limiting its scope, we will further illustrate the invention by discussing the alkylation of saturated hydrocarbons, in general branched saturated hydrocarbons, with an olefin to give highly branched saturated hydrocarbons with a higher molecular weight.

This reaction is of interest to the petroleum industry because it makes it possible to obtain, through the alkylation of isobutane with an olefin containing 2-6 carbon atoms, an alkylate which has a high octane number and which boils in the gasoline range. Unlike gasoline obtained by cracking heavier petroleum fractions such as vacuum gas oil and atmospheric residue, gasoline obtained by alkylation is essentially free of contaminants such as sulphur and nitrogen and so has clean burning characteristics. Its high anti-knock properties, represented by high octane numbers, lessen the need to add environmentally harmful anti-knock compounds such as aromatics or lead. Also, unlike gasoline obtained by reforming naphtha or by cracking heavier petroleum fractions, gasoline obtained by alkylation contains few, if any, aromates or olefins, which, environmentally speaking, is a further advantage.

US 5,073,665 discloses an alkylation process using a reactor containing multiple fixed beds. Each of these beds can have its own supply of olefin and the reactor effluent can be recycled, leading to a high isoparaffin to olefin weight ratio (hereinafter: i/o ratio) in the reactor.
The disadvantage of this process is that its efficiency is not very high, because of inhomogeneous deactivation of the catalyst in fixed beds. Initially, when the catalyst is fresh, high reaction rates are observed at the most upstream part of a fixed bed, with consequently lower reaction rates in the more downstream parts. This gradient in reaction rate will lead to a gradient in deactivation rate of the catalyst: the most upstream part will deactivate faster than more downstream part. As a result, the highest reaction rate shifts to more downstream parts of the bed.

On the other hand, US 5,811,626 mentions the use of a liquid fluidized catalyst bed in an alkylation process. The advantage of liquid fluidized beds is that the catalyst particles move freely through the bed, leading to a more uniform deactivation of the entire catalyst bed.

The present process combines the advantages of these prior art alkylation processes by using a reactor containing at least two catalyst beds, at least one of them being liquid fluidized during at least a part of the process. The process involves the following steps:
(a) introducing alkylatable compound into the reactor at or near the most upstream catalyst bed,
(b) introducing alkylation agent into said reactor through at least two injection points,
(c) reacting a portion of the alkylatable compound with at least a portion of the alkylation agent to produce an alkylate-containing product stream, and
(d) circulating at least a portion of the alkylate-containing product stream to the most upstream catalyst bed of said reactor.

With this process, a high i/o ratio can be reached, combined with a more uniform deactivation of the catalyst beds.
That this combination is not obvious is because in order to reach a satisfying degree of fluidization the feed must have a relatively high linear velocity. However, this velocity must not be too high, as this will lead to disintegration of the catalyst bed. In case of an upflow reactor with multiple liquid fluidized beds and multiple injection points of alkylation agent, the linear velocity of the feed increases while flowing from bottom to top. Hence, one would fear that the linear velocity in such reactors would become too high to perform the process successfully.

Preferably, the catalyst is regularly regenerated with hydrogen. As will be explained below, this is easily performed by switching the reactor between an alkylation and a regeneration mode.
Therefore, in one embodiment of the invention, the catalyst beds in the reactor are present as fixed beds while being in the alkylation mode, whereas during regeneration at least one of these beds is liquid fluidized. This can be performed by working downflow (i.e. flowing alkylatable compound from top to bottom) during the alkylation mode, while working upflow during the regeneration mode. So, this results in a process wherein at least one of the catalyst beds is liquid fluidized during a part of the process, i.e. the regeneration part.
According to another embodiment, at least one of the catalyst beds is liquid fluidized during both the alkylation and the regeneration modes, i.e. during the the entire process.

The reactor comprises at least two catalyst beds. Preferably, the reactor comprises 2-10 catalyst beds, more preferably 2-6. At least one of these beds is a liquid fluidized catalyst bed during at least a part of the process. Preferably, at least two of these beds are liquid fluidized during at least a part of the process. More preferably, all of these beds are liquid fluidized during at least a part of the process.
The catalyst beds that are not liquid fluidized are generally fixed beds.

In a liquid fluidized bed the linear velocity of the liquid is above the minimum required for the fluidization of the catalyst particles. The required linear velocity depends, amongst others, on the particle size, catalyst density, and liquid velocity.

The actual linear velocity at a given point in the reactor depends on the velocity of the alkylate-containing product stream and the amount of alkylation agent added. Hence, the lower the total amount of alkylation agent relative to the alkylating product stream, the lower the increase in linear velocity.
Preferably, the linear velocity is such that the liquid fluidized catalyst bed height increases 0-60% relative to the height of the liquid fluidized catalyst bed operating beneath the minimum fluidization velocity. More preferably, this increase is 5-50%, even more preferably 10-30%

Not every catalyst bed has to have the same size, shape, and/or composition. For instance, the most downstream catalyst bed can have a larger size than the more upstream bed(s) to ensure complete conversion of the alkylation agent. Furthermore, the different beds can comprise different catalysts.
In order to obtain at the same time both fixed and liquid fluidized beds in a reactor, some of the beds may consist of smaller particles than other beds. Depending on these particle sizes and the linear velocity, the catalyst beds containing the small particles can be liquid fluidized while the beds containing larger particles remain fixed.

Alkylation agent is introduced via more than one injection point. In one embodiment, each bed is provided with its own addition of alkylation agent. In another embodiment, the most downstream catalyst bed is deprived from such addition. This ensures complete conversion of the alkylation agent in the reactor.

At least a portion of the alkylate-containing product stream (containing alkylate and alkylatable compound) that leaves the reactor is circulated to the most upstream catalyst bed of the reactor. This portion preferably ranges from 60 to 98%, more preferably 80 to 90% of the entire product stream.
The rest of the alkylate-containing product stream is transported to, e.g., a subsequent reactor or a separation unit wherein alkylatable compound is separated from the product stream.

Within this specification the term 'most upstream catalyst bed' refers to the first catalyst bed the circulated alkylate-containing product stream passes after entering the reactor.
The portion of the alkylate-containing product stream that is recycled to the reactor can be made up with alkylatable compound. At least a fraction of this portion is recycled to the most upstream catalyst bed of the reactor. It is possible to recycle other fractions to one or more of the other catalyst beds present in the reactor.

The size of the product stream and the amount of alkylation agent added to each catalyst bed depends on the desired i/o ratio in the bed.
The desired i/o ratio generally ranges between 50 and 5000, more preferably between 200 and 2000, and most preferably between 400 and 1000.

The process is typically performed under conditions such that at least a portion of the alkylation agent and the alkylatable compound are in the liquid phase or the supercritical phase. In general, the process according to the invention is performed at a temperature in the range of 233 to 523 K, preferably in the range of 293 to 423 K, more preferably in the range of 338 to 368 K, and a pressure in the range 1 to 100 bar, preferably 5 to 40 bar, more preferably 15 to 30 bar.

The catalyst used in the process according to the invention preferably comprises a hydrogenating metal component and a solid acid constituent.
Examples of suitable hydrogenating metal components are constituents of the transition metals, such as metals of Group VIII of the Periodic Table, or mixtures thereof. Among these, noble metals of Group VIII of the Periodic Table are preferred. Platinum, palladium, and mixtures thereof are especially preferred. The amount of hydrogenating metal component will be dependent on its nature. When the hydrogenating metal component is a noble metal of Group VIII of the Periodic Table, the catalyst generally will contain in the range of 0.01 to 2 wt.% of the metal, preferably 0.1-1 wt.%, calculated as metal.

Examples of solid acid constituents are zeolites such as zeolite beta, MCM-22, MCM-36, mordenite, X-zeolites and Y-zeolites, including H-Y-zeolites and USY-zeolites, non-zeolitic solid acids such as silica-alumina, sulphated oxides such as sulphated oxides of zirconium, titanium, or tin, sulphated mixed oxides of zirconium, molybdenum, tungsten, etc., and chlorinated aluminium oxides. The presently preferred solid acid constituents are zeolites, including mordenite, zeolite beta, X-zeolites and Y-zeolites, including H-Y-zeolites and USY-zeolites, sulphated oxides, and chlorinated aluminium oxides. Mixtures of solid acid constituents can also be employed.

The catalyst preferably comprises a hydrogenating metal component on a carrier which comprises 2-98 wt.% of solid acid constituent and 98-2 wt.% of a matrix material, calculated on the carrier. More preferably, the carrier comprises 10-90 wt.% of matrix material, and 90-10 wt.% of solid acid constituent. More preferably, the carrier comprises 10-80 wt.% of matrix material and the balance is solid acid constituent. Especially preferred is a catalyst wherein the carrier comprises 10-40 wt.% of matrix material and the balance is solid acid constituent.
In the present specification the term matrix material encompasses all components which are present in the catalyst except for the solid acid constituent and the hydrogenating metal component. Examples of suitable matrix materials are alumina, silica, clays, and mixtures thereof. Matrix materials comprising alumina are generally preferred. A matrix material which consists essentially of alumina is considered most preferred at this point in time.

Preferably, the catalyst to be used in the process according to the invention has a particle size of at least 0.5 mm. Preferably, the particle size is at least 0.8 mm, more preferably at least 1.0 mm. The upper limit of the particle size preferably lies at 10 mm, more preferably at 5 mm, even more preferably at 3 mm.
In the present specification, the term particle size is defined as the average diameter of the solid part of the catalyst.

The catalyst can be prepared by processes common to the industry. These will comprise shaping the solid acid constituent after mixing it with a matrix material, to form particles, followed by calcination of the particles. The hydrogenating function may be incorporated into the catalyst composition by impregnating the carrier particles with a solution of a hydrogenation metal component.

As mentioned above, the catalyst may have to be regenerated regularly with hydrogen. Preferably, two types of regeneration are used: mild regeneration and high temperature regeneration. Mild regeneration is generally carried out at a temperature in the range of 233 to 523 K and a pressure from 1 to 100 bar, whereas high-temperature regeneration is usually effected at a temperature of at least 423 K, preferably in the range of 423-873, more preferably 473-673 K.
Mild regeneration of the catalyst is performed by contacting the catalyst with hydrogen in the presence of hydrocarbon, e.g. the product stream, alkylatable compound, or formed alkylate already present in the reactor. Typically, the hydrogen will be dissolved in the hydrocarbon. Preferably, the solution contains at least 10% of the saturation concentration of hydrogen; said saturation concentration being defined as the maximum quantity of hydrogen which can be dissolved in the hydrocarbon at regeneration temperature and pressure. Depending on the applied feed rates it may be more preferred for the solution to contain at least 50% of the saturation concentration, even more preferably at least 85%. At relatively low feed rates it is generally preferred to have as saturated a solution of hydrogen in the mixture as possible.

The regeneration frequency depends on a number of conditions, including the nature of the catalyst, the reaction and regeneration conditions, and the amount of hydrogen present during the regeneration step. Preferably, mild regeneration is performed before there is any substantial decrease of catalytic activity. Such a decrease can be observed by breakthrough of alkylation agent, which can be measured by analysing the concentration of alkylation agent in the product stream. If mild regeneration is performed prior to breakthrough of alkylation agent it is possible to obtain a product of nearly constant composition in a high yield. Typically, the mild regeneration frequency is in the range of once per 10 hours to 10 times per hour, preferably once per 3 hours to 3 times per hour and even more preferably once per 2 hours to 2 times per hour.
To effect a long-term process on a commercial scale one can carry out a high-temperature regeneration after every 50, preferably after every 100 mild regenerations. Pilot plant experiments have shown that it is possible to effect a long-term process when the catalyst is subjected to a high-temperature regeneration after every 200-400 mild regenerations. Depending on the exact process variables on a commercial scale, this value may be higher or lower in actual practice.

In a preferred embodiment the process is performed using at least two reactors. During operation at least one of these reactors is/are used for the alkylation; this/these reactor(s) is/are said to be in the alkylation mode. In the other reactor(s) the catalyst is mildly regenerated. This/these reactor(s) is/are said to be in the regeneration mode. Regularly, the reactors are switched from alkylation to mild regeneration mode and *vice versa.* In this way, the entire process of alkylation and regeneration can be performed continuously.
In an even more preferred embodiment, at least four reactors are used: at least two in the alkylation mode and at least two in the regeneration mode.

Additionally, one or more additional reactors may be used that can replace one or more of the reactors in the alkylation or regeneration mode. The availability of such additional reactor(s) makes the process very flexible. If, owing to circumstances, the catalyst in one or more of the reactors deactivates to an unacceptable extent during the process, the reactor(s) can be replaced with one or more additional reactors. The deactivated catalyst can then be regenerated by contacting it at high temperature with hydrogen in the gas phase to recover its original activity without affecting the ongoing alkylation process. After this high-temperature regeneration the reactor containing the high-temperature regenerated catalyst can serve as said additional reactor.

Most preferably, this high-temperature regeneration is preceded by a mild regeneration. This mild regeneration is preferably performed for a longer period of time than mild regeneration during the regeneration mode.

Preferred alkylatable compounds to be used in the process according to the invention are isoalkanes having 4-10 carbon atoms, such as isobutane, isopentane, isohexane, or mixtures thereof.
Preferred alkylation agents are olefins having 2-10 carbon atoms, preferably 2-6 carbon atoms, more preferably 3-5 carbon atoms, e.g. propene, butene, pentene. The alkylation of isobutane with butene or a mixture of butenes constitutes an attractive embodiment of the process according to the invention.

If mild regeneration is performed, it is generally carried out at a temperature in the range of 233 to 523 K and a pressure from 1 to 100 bar. Although regeneration and alkylation steps can be performed at different temperatures and pressures, it is preferred for the regeneration temperature, expressed in K, and the regeneration pressure not to differ from the reaction temperature and pressure by more than 20%, more preferably not by more than 10%, still more preferably not by more than 5%. Most preferably, the regeneration temperature and pressure and the reaction temperature and pressure are essentially the same.

Preferred alkylates to be produced by the process according to the invention are C₅+ alkylates with a minimum of C₉+ alkylates. The C₅+ alkylate obtained using the process according to the invention preferably has a C₉+ content of less than 30 wt.%, more preferably of less than 20 wt.%, most preferably of less than 10 wt.%. Frequent mild regeneration enables C₉+ production to be controlled at a comparatively low level.
Also, depending on the regeneration frequency, in the process according to the invention a high C₅+ alkylate yield is obtained. The process according to the invention makes it possible to obtain a C₅+ alkylate yield in excess of 200%, calculated on the weight of the consumed olefin, preferably of 204% or higher.

The quality of the alkylate product obtained in the process according to the invention can be measured by the RON of the product. The RON is a measure of the anti-knock rating of gasoline and/or gasoline constituents. The higher the RON, the more favourable the anti-knock rating of the gasoline will be. Depending on the type of gasoline engine, generally speaking a higher anti-knock rating is of advantage when it comes to the working of the engine. The product obtained in the process according to the invention preferably has a RON of 90 or higher, more preferably of 92 or higher, most preferably 94 or higher. The RON is obtained by determining, e.g., via gas chromatography, the percentage by volume of the various hydrocarbons in the product. The percentages by volume are then multiplied by the RON contribution and added up.
Examples of compounds with a RON of 90 or higher are isopentane, 2,2-dimethyl butane, 2,3-dimethyl butane, trimethyl butane, 2,3-dimethyl pentane, 2,2,4-trimethyl pentane, 2,2,3-trimethyl pentane, 2,3,4-trimethyl pentane, 2,3,3-trimethyl pentane, and 2,2,5-trimethyl hexane.

## Claims

1. Process for the reaction of an alkylatable compound and an alkylating agent using a reactor containing at least two catalyst beds, at least one of them being liquid fluidized during at least a part of the process, the process involving the following steps:
(a) introducing alkylatable compound into the reactor at or near the most upstream catalyst bed,
(b) introducing alkylation agent into said reactor through at least two injection points,
(c) reacting a portion of the alkylatable compound with at least a portion of the alkylation agent to produce an alkylate-containing product stream, and
(d) circulating at least a portion of the alkylate-containing product stream to the most upstream catalyst bed of said reactor.

2. Process according to claim 1 wherein the process involves a catalyst regeneration step.

3. Process according to any one of the preceding claims wherein the reactor comprises at least two liquid fluidized catalyst beds during at least a part of the process.

4. Process according to any one of the preceding claims wherein the most downstream catalyst bed is larger than the upstream catalyst bed or beds.

5. Process according to any one of the preceding claims wherein the at least two catalyst beds do not all contain the same catalyst.

6. Process according to any one of the preceding claims wherein an injection point is located at or near each of the at least two liquid fluidized catalyst beds.

7. Process according to claim 6 wherein alkylation agent is introduced through each of the injection points.

8. Process according to any one of claims 1-6 wherein no alkylation agent is introduced at or near the most downstream catalyst bed.

9. Process according to any one of the preceding claims wherein 60-98% of the alkylate-containing product stream is circulated to the most upstream catalyst bed.

10. Process according to any one of the preceding claims wherein the isoparaffin to olefin ratio ranges from 50 to 5000.
